# FASCICULE DE BREVET EUROPEEN

(11) **EP 2 466 287 B1**
(45) Date de publication et mention de la délivrance du brevet: **18.10.2017**
(21) Numéro de dépôt: 11193861.9
(22) Date de dépôt: 15.12.2011
(51) Int. Cl.: G01M 3/20, G01N 33/00

(54) **Dispositif et procédé de détection de fuite utilisant l'hydrogène comme gaz traceur**
Vorrichtung und Verfahren zum Erkennen von Lecks mit Hilfe von Wasserstoff als Spürgas
Device and method for detecting leaks using hydrogen as a tracer gas

(30) Priorité: 17.12.2010 FR 1060729
(43) Date de publication de la demande: 20.06.2012
(73) Titulaire: Pfeiffer Vacuum, 74000 Annecy (FR)
(72) Inventeur: Patel, Ketan, Monroeville PA-15146 (US); Rouveyre, Frédéric, 74000 Annecy (FR)
(74) Mandataire: Croonenbroek, Thomas Jakob

(56) Documents cités:
- WO-A1-97/39322
- WO-A1-2005/054806
- CN-U- 2 051 351
- DE-A1- 19 853 049
- US-A- 3 939 695
- US-A- 5 293 771
- US-A- 5 932 797
- US-B1- 6 314 793
- US-B1- 7 389 675

## Description

La présente invention se rapporte à un dispositif de détection de grosses fuites, par exemple allant de quelques Pa.m³/s jusqu'à 10-⁶ Pa.m³/s, par la technique d'aspersion (« spraying » en anglais).

La technique de détection de fuite par aspersion consiste à placer l'objet à tester dans une enveloppe étanche dont l'atmosphère contient majoritairement un gaz traceur. On évacue le gaz intérieur de l'objet jusqu'à une pression faible, par exemple de l'ordre de 10 hPa. On recherche alors la présence de grosses fuites en détectant la présence ponctuelle de gaz traceur à l'intérieur de l'objet, placé dans l'enveloppe de test, à l'aide d'un détecteur de fuites. L'atmosphère interne de l'objet à tester est reliée à un détecteur de gaz traceur. Plusieurs méthodes de détection de fuite sont connues.

Une première méthode utilise l'hélium comme gaz traceur et un spectromètre de masse comme détecteur. On utilise de cette façon la propriété de l'hélium qui traverse les petites fuites plus aisément que les autres gaz, du fait de la petite taille de sa molécule. Cette méthode est donc d'une grande sensibilité et elle est habituellement utilisée pour la détection de fuites allant de 10⁻⁵ à 10⁻¹² Pa m³ / s. Cependant la méthode à l'hélium présente comme inconvénients le coût élevé du gaz traceur et la nécessité de faire le vide à l'intérieur de l'objet à tester afin de permettre le fonctionnement du spectromètre de masse. Pour maintenir une pression très faible, un groupe de pompage gros et coûteux est nécessaire. En outre, lorsque l'objet à tester est de grand volume, il peut être difficile, voire impossible, d'atteindre le niveau de pression requis. Et si le niveau de pression requis peut être atteint, le cycle de pompage est très long. Lorsque le taux de fuite est très élevé, il y a un risque que des dégâts soient occasionnés dans le spectromètre de masse. Le filament du spectromètre de masse peut être pollué et même se rompre. Enfin l'entretien d'un système aussi compliqué est très coûteux.

Pour parer à ces inconvénients, une méthode utilisant l'hydrogène comme gaz traceur a été développée. L'hydrogène présente l'avantage d'une vitesse de diffusion beaucoup plus rapide à l'intérieur des objets en test et se dissipe plus rapidement que l'hélium. Il est beaucoup moins onéreux que l'hélium et la détection est réalisée par différents types de capteurs fonctionnant à pression atmosphérique. De ce fait cette méthode constitue une alternative très intéressante pour l'industrie car moins onéreuse et plus simple à mettre en oeuvre. Toutefois cette méthode présente une sensibilité moindre par rapport à la méthode à l'hélium, ce qui limite son utilisation. Des méthodes analogues sont décrites dans les documents US5932797 et DE19853049. La présente invention a pour but de proposer un dispositif de détection de fuite permettant de détecter des fuites importantes, jusqu'à 10⁻⁶ Pa.m³/s avec une bonne sensibilité.

L'invention propose aussi un dispositif de détection de fuite robuste, léger et peu coûteux.

L'objet de la présente invention est un dispositif de détection de fuite au moyen d'hydrogène comme gaz traceur, destiné à être relié à un objet à tester, comprenant un capteur d'hydrogène, placé dans une enceinte basse pression comprenant une diode, une résistance, et un transistor de type MOS dont la grille est recouverte d'un catalyseur au palladium, un moyen de pompage reliée à l'enceinte basse pression, une jauge de pression configurée pour mesurer la pression dans une ligne de vide formée par l'enceinte basse pression reliée au moyen de pompage, et une vanne multivoies comprenant une première voie permettant l'admission du flux gazeux contenant le gaz traceur dans la ligne de vide et une deuxième voie permettant l'admission de gaz neutre, comportant un module de contrôle électronique de la vanne multivoies configurée pour contrôler la pression de la ligne de vide en fonction de la jauge de pression par le contrôle de l'admission de gaz neutre dans la ligne de vide à travers la deuxième voie d'admission. On entend par vanne multivoies, tout moyen comprenant une ou plusieurs vannes configurée pour contrôler la communication entre au moins trois voies, une première voie communicant avec l'objet à tester, une deuxième voie communicant avec le capteur et au moins une troisième voie pour l'admission d'air par exemple.

Selon un premier mode de réalisation, la deuxième voie est configurée pour s'ouvrir sur l'atmosphère extérieure. Selon un deuxième mode de réalisation, le dispositif comprend en outre un filtre à particules interposé entre le capteur et la vanne multivoies pour filtrer les particules de poussière contenues dans le gaz avant que celui-ci n'entre en contact avec le capteur d'hydrogène.

Selon un troisième mode de réalisation, le dispositif comprend en outre un module de contrôle électronique configuré pour recevoir la valeur de la pression de la ligne de vide mesurée par la jauge de pression et recevoir une valeur consigne et configurée pour piloter l'admission de gaz neutre en fonction de ces valeurs.

Selon un quatrième mode de réalisation, le dispositif comprend en outre un modèle mathématique pour calculer le taux de fuite à partir de la variation de la tension drain - source du transistor contenu dans le capteur en fonction du temps.

Selon un cinquième mode de réalisation, le dispositif comprend en outre un circuit d'équilibrage de la tension de la résistance du capteur.

Avantageusement le circuit d'équilibrage de la tension de la résistance du capteur est configuré pour maintenir une température fixe indépendamment de la valeur de la pression dans l'enceinte basse pression.

La température fixe est comprise par exemple entre 100°C et 250°C.

La pression dans l'enceinte basse pression est comprise par exemple entre 100 Pa et 5000 Pa.

L'invention a aussi pour objet un procédé de détection de fuite d'un objet à tester au moyen d'un dispositif de détection de fuite tel que décrit précédemment, dans lequel on contrôle la pression de la ligne de vide en contrôlant l'admission du gaz neutre dans la ligne de vide à travers la deuxième voie d'admission de la vanne multivoie.

Selon un mode réalisation, on contrôle l'admission du gaz neutre en pilotant la deuxième voie de manière automatique en fonction de la mesure de la pression et de la valeur de la consigne prédéterminée.

La présente invention présente de nombreux avantages. Le dispositif de détection a une très grande sensibilité à très basse pression pour des taux de fuite jusqu'à 10⁻⁶ Pa.m³/s. Le capteur d'hydrogène est utilisable sous une basse pression, comprise entre 100 Pa et 5000 Pa, par exemple 1000 Pa (10 mbar), pression qui est facilement atteinte avec une petite pompe à vide à membrane par exemple, et ne nécessite pas de groupe de pompage de grande puissance, coûteux et encombrant. Le capteur d'hydrogène est bon marché à fabriquer et possède une longue durée de vie. Si nécessaire il peut être facilement remplacé. Ce capteur d'hydrogène permet au dispositif de détection d'être léger et peu encombrant, et donc facilement transportable. Il est facilement intégrable dans des appareillages existants comportant un dispositif de détection de fuite.

L'invention peut s'appliquer à tous types d'objet, mais plus spécialement aux chambres utilisées en fabrication dans l'industrie ne nécessitant pas un vide très poussé, par exemple des fours sous vide pour le traitement des matériaux ou le dépôt de revêtement (pour des verres de lunettes de soleil par exemple).

D'autres caractéristiques et avantages de la présente invention apparaîtront à la lecture de la description qui suit d'un mode de réalisation, donné bien entendu à titre illustratif et non limitatif, et dans le dessin annexé sur lequel
- la figure 1 illustre un premier mode de réalisation d'un dispositif de détection de fuite en configuration de détection,
- la figure 2 illustre le procédé permettant de stabiliser la pression de la ligne de vide,
- la figure 3 illustre un mode de réalisation d'un capteur d'hydrogène,
- la figure 4 illustre un exemple de dispositif de test destiné à évaluer la sensibilité d'un capteur d'hydrogène,
- la figure 5 illustre le résultat d'un test de sensibilité d'un capteur d'hydrogène en fonction de la pression dans le dispositif de test, la tension V de la résistance du capteur d'hydrogène en Volts est portée en ordonnée et en abscisse la pression P en mbar,
- la figure 6 illustre le résultat d'un test de sensibilité du capteur d'hydrogène pour différentes valeurs du taux de fuite et de la pression dans le dispositif de test,
- la figure 7 illustre le résultat d'un test de sensibilité du capteur d'hydrogène pour différentes distances entre la fuite et le capteur d'hydrogène et différents taux de fuite,
- la figure 8 illustre un autre exemple de dispositif de test destiné à évaluer la sensibilité d'un capteur d'hydrogène, en fonction de sa position par rapport au flux de gaz de test,
- la figure 9 illustre le résultat d'un test de sensibilité du capteur d'hydrogène pour différentes positions dans le dispositif de détection de fuite.

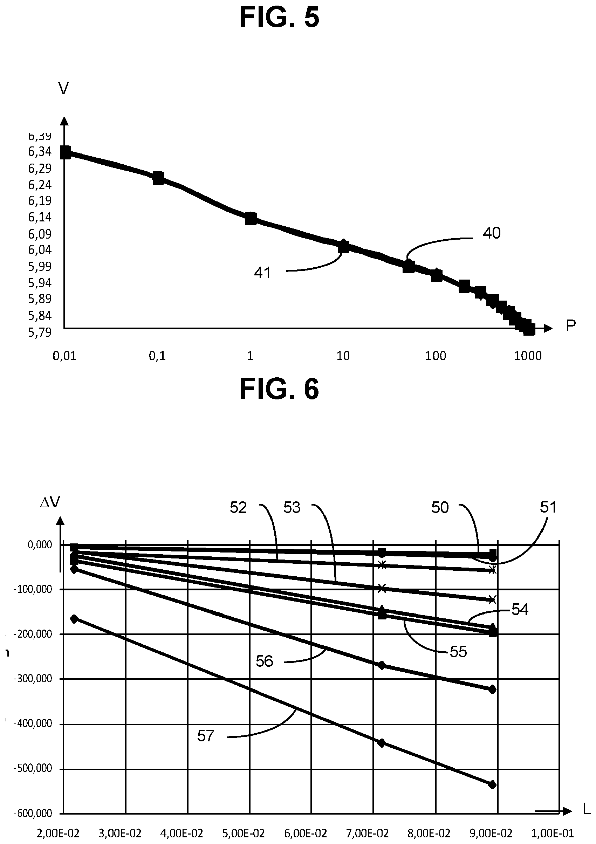

Sur les figures 6, 7 et 9, en ordonnée est portée la variation de la tension ΔV dans le temps en mV / s en fonction du taux de fuite L en mbar.l.s⁻¹ en abscisse.

Sur ces figures, les éléments identiques portent les mêmes numéros de référence.

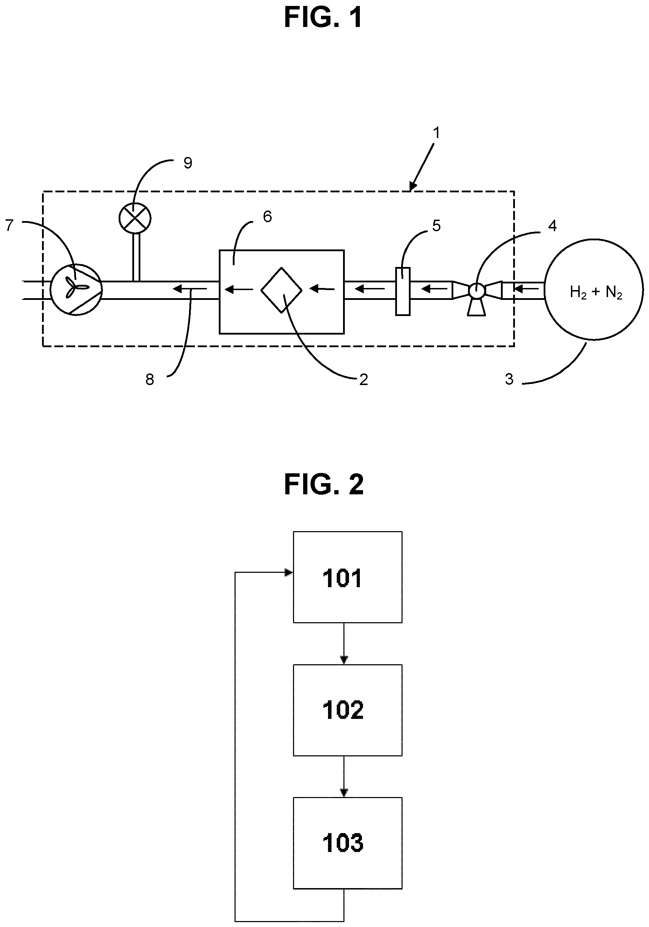

Dans le mode de réalisation illustré sur la figure 1, le dispositif de détection **1** de fuite comprend un capteur **2** d'hydrogène relié à l'atmosphère intérieure d'un objet à tester **3.** Le dispositif de détection **1** comprend une vanne multivoies, qui peut être notamment une vanne **4** à trois voies, contrôlant la mise en communication du capteur **2** d'hydrogène avec l'objet à tester **3** pour permettre l'admission du flux gazeux contenant le gaz traceur dans le dispositif **1** de détection de fuite, et un filtre **5** à particules interposé entre le capteur **2** et la vanne **4** à trois voies pour filtrer les particules de poussière contenues dans le gaz avant que celui-ci n'entre en contact avec le capteur **2** d'hydrogène. La présence du filtre **5** permet de protéger le capteur **2** et de prolonger sa durée de vie. Selon une variante, la vanne **4** à trois voies peut être utilisée pour réguler l'entrée d'air afin de maintenir une basse pression, comprise entre 100 Pa et 5000 Pa, comme par exemple 1000 Pa, dans la ligne de vide formée par une enceinte basse pression **6** reliée à un moyen de pompage **7**. .Alternativement la vanne **4** à trois voies peut être disposée après le capteur.

Le capteur **2** d'hydrogène est placé dans l'enceinte basse pression **6** reliée au moyen de pompage **7** qui permet d'une part d'atteindre et de maintenir une basse pression dans la enceinte basse pression **6**, par exemple 1000 Pa, et d'autre part de créer un flux de gaz **8** dans le dispositif de détection **1.** Le moyen de pompage **7,** permettant d'abaisser la pression, est placé en aval du capteur **2** d'hydrogène, du côté opposé à l'arrivée du gaz provenant de l'objet à tester **3,** afin de créer une circulation du gaz **8.** Ainsi il n'y a ainsi aucune accumulation de gaz en amont du capteur **2,** et seules les molécules d'hydrogène provenant de la fuite à mesurer entrent en contact avec le capteur **2** d'hydrogène. Cette configuration a pour avantage d'augmenter le temps de réponse du capteur **2.** De préférence le capteur **2** d'hydrogène est disposé de manière à ce que la tête du capteur **2** se trouve face au flux de gaz **8** contenant de l'hydrogène issu de l'objet à tester **3** et pompé par le moyen de pompage **7** de manière à augmenter la sensibilité du capteur **2** d'hydrogène. Le moyen de pompage **7** peut être de tout type, comme une pompe à vide, par exemple une pompe à vide à membrane ; en particulier on choisira de préférence une pompe à vide à membrane de référence « AMD4 » commercialisée sous la marque «ADIXEN». Une jauge de pression **9,** telle qu'un manomètre, montée sur le conduit reliant l'enceinte basse pression **6** et le moyen de pompage **7,** permet de contrôler la pression atteinte dans l'enceinte basse pression **6.**

Pour effectuer la mesure de la fuite, l'entrée du dispositif de détection **1** est relié à l'objet à tester **3.** Le moyen de pompage **7** abaisse la pression dans la ligne de vide. La vanne **4** à trois voies est alors progressivement ouverte. Le dispositif de détection de fuite comporte un module de contrôle électronique configuré pour recevoir la valeur de la pression de la ligne de vide mesurée par la jauge de pression **9,** configuré pour recevoir une valeur consigne et configuré pour piloter l'admission de gaz neutre en fonction de ces valeurs. Le module de contrôle électronique permet de s'assurer qu'une pression stable, par exemple 1000 Pa, est atteinte dans la ligne de vide, ceci afin d'éviter les fluctuations dans la mesure de la fuite. La mesure peut alors commencer. L'extérieur de l'objet à tester **3** est aspergé d'un gaz, composé par exemple d'un mélange de 95% d'azote et de 5% d'hydrogène. Sous l'effet du pompage, une partie de ce gaz traverse la paroi de l'objet à tester **3** au niveau de la fuite et entre dans le dispositif de détection **1** de fuite. L'aspersion de gaz s'effectue étape par étape sur chaque zone de la surface extérieure de l'objet à tester **3,** avec un temps d'attente de quelques secondes entre chaque étape afin de laisser le temps au gaz pompé d'atteindre le capteur **2** d'hydrogène.

Un moyen électronique de régulation de la température peut être ajouté au dispositif de détection de fuite de sorte que la température n'influe pas sur la pression.

Des étapes du procédé permettant de maintenir une pression stable sont représentées sur la figure 2. Dans une première étape **101,** la pression de la ligne de vide est mesurée grâce à la jauge de pression **9.** Dans une deuxième étape **102** le module de contrôle électronique compare la valeur de pression mesurée par la jauge **9** avec une valeur de pression consigne. Dans une troisième étape **103,** le module de contrôle électronique pilote l'ouverture ou la fermeture deuxième voie d'admission de gaz afin que la pression de la ligne de vide atteigne la valeur de pression consigne. Ce procédé est répété en boucle pendant la mesure de fuite. L'admission de gaz est faite de manière automatique en fonction de la pression mesurée et de la valeur de pression consigne prédéterminée.

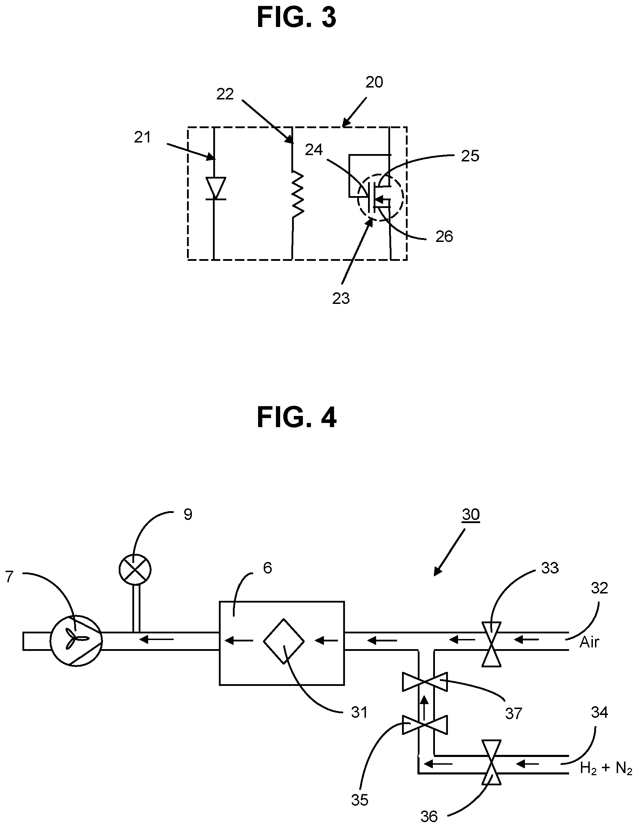

On considérera maintenant la figure 3 qui illustre un mode de réalisation d'un capteur **20** d'hydrogène. Le capteur **20** comporte une diode **21,** une résistance **22** et un transistor **23** qui sont interconnectés. Le transistor **23** est un transistor à effet de champ de type MOSFET ("Metal Oxide Semiconductor Field Effect Transistor" en anglais, qui se traduit par transistor à effet de champ à structure métal-oxyde-semiconducteur). Le transistor **23** est de type « N » et comporte trois électrodes actives, la grille **24,** le drain **25** et la source **26.** La grille **24** (« gate » en anglais) est reliée au drain **25.** Le transistor **23** module le courant qui le traverse au moyen d'un signal appliqué sur la grille **24,** permettant de contrôler la tension entre le drain **25** et la source **26.** La grille **24** est recouverte d'un catalyseur à base de palladium. Lorsque la molécule d'H₂ arrive au contact du catalyseur au palladium de la grille **24,** elle est brisée pour donner des ions H⁺. Les ions H⁺ diffusent à travers le catalyseur et se retrouve piégés sur la grille **24** du transistor **23.** Ceci cause une variation de la résistance drain **25 -** source **26** du transistor **23.** La résistance drain **25 -** source **26** du transistor **23** constitue ainsi le coeur du capteur assurant la fonction de mesure de la fuite. En injectant un courant constant dans le drain **25** du transistor **23,** on obtient une tension V représentant la quantité d'ions H⁺ piégée par la grille **24** qui permet d'évaluer le flux d'hydrogène arrivant au contact de la grille **24.**

Un module de contrôle électronique comprend notamment des circuits électroniques pour piloter le dispositif de détection de fuite, et un modèle mathématique pour calculer le taux de fuite. Le modèle mathématique a été développé pour donner une mesure du taux de fuite à partir de la variation de la tension drain **25 -** source **26** du transistor **23** en fonction du temps, liée à la sensibilité à l'hydrogène du capteur **20.** Il est possible d'ajouter une option de calibrage automatique, avec un taux de fuite fixe, basé sur ce modèle mathématique. Le nombre d'atomes d'hydrogène qui frappe la surface active de la grille **24** du transistor **23** étant proportionnel à la pression dans l'enceinte basse pression **6,** la résistance drain **25 -** source **26** sera donc proportionnelle à la pression absolue d'hydrogène autour du transistor **23** dans l'enceinte basse pression **6.** A une pression résiduelle de 1000 Pa, une monocouche d'atomes d'hydrogène se forme en environ 25 µs.

Une résistance **22** chauffante permet de chauffer le capteur **20** d'hydrogène à une température de 130°C par exemple. Une température de cet ordre augmente avantageusement la sensibilité du capteur **20** d'hydrogène. Toutefois la température ne doit pas excéder 250°C, cette limite étant imposée par le silicium du transistor **23.** Des essais ont montré qu'une température de 180°C peut être utilisée sans dommages. A titre d'exemple, la résistance chauffante du capteur **20** d'hydrogène peut avoir une valeur comprise entre 70 Ω et 80 Ω ; à pression atmosphérique, le courant de chauffage sera de 60 à 80 mA, soit une puissance de chauffe de 0,4 W approximativement.

Une diode **21** est utilisée pour mesurer la température du capteur **20** d'hydrogène qui peut être alimentée par un courant de 1 mA lorsque la tension à ses bornes atteint 590 mV. Cette valeur de la tension correspond à une température de 130°C. Le coefficient de température de la diode est de -1,6 mV/K.

Pour des raisons de sécurité, on utilise un mélange gazeux composé de 95% d'azote et de 5% d'hydrogène qui n'est mis en contact avec le capteur **20** d'hydrogène qu'une fois la température de 130°C atteinte pour éviter toute dégradation du catalyseur. C'est également pour préserver le catalyseur que le capteur **20** d'hydrogène est utilisé sous très basse pression afin de n'être en contact qu'avec de faible quantité d'hydrogène.

Après la mesure, le capteur **20** d'hydrogène peut être nettoyé par injection d'un mélange gazeux contenant 5% d'hydrogène pendant environ 10 secondes, une augmentation de température favorisant la récupération du catalyseur après saturation.

La figure 4 illustre un dispositif de test **30** destiné à évaluer la sensibilité d'un capteur **31** d'hydrogène à la pression dans le dispositif de test **30.** Le dispositif de test **30** comporte un capteur **31** d'hydrogène placé dans une enceinte basse pression **6** reliée à un moyen de pompage **7** par un conduit, l'ensemble enceinte basse pression et conduit formant une ligne de vide. La pression dans la ligne de vide étant pilotée à partir de la mesure réalisée par une jauge de pression **9.** Le dispositif de test **30** comporte une entrée d'air **32** contrôlée par une vanne d'admission **33.** Le dispositif de test **30** comporte aussi une entrée de gaz traceur **34,** ici un mélange gazeux composé de 95% d'azote et de 5% d'hydrogène, dont le flux est régulé par une microvanne **35.** Une première vanne d'isolement **36** et une seconde vanne d'isolement **37,** disposées de part et d'autre de la microvanne **35,** sont manoeuvrées manuellement pour autoriser ou interrompre le flux de gaz traceur. L'entrée d'air **32** et l'entrée de gaz traceur **34** communiquent par un conduit commun avec l'enceinte basse pression **6** contenant le capteur **31** d'hydrogène.

Dans un premier temps on a évalué la sensibilité à la pression du capteur **31** d'hydrogène dans la ligne de vide. La ligne de vide est maintenue à basse pression, par exemple 1000 Pa, grâce au moyen de pompage **7** d'une part et à la jauge de pression **9** d'autre part qui pilote la vanne d'admission **33** permettant l'introduction d'air si nécessaire pour rétablir la pression à la valeur fixée. La résistance du capteur 31 est utilisée pour chauffer le capteur **31** d'hydrogène à 130°C afin d'améliorer sa sensibilité.

La figure 5 illustre la sensibilité de la tension de la résistance du capteur **31** en fonction de la pression dans l'enceinte basse pression **6.** On observe une bonne reproductibilité de la mesure et une grande sensibilité du capteur **31** d'hydrogène à la pression, les courbes ascendantes **40** et descendantes **41** étant confondues. Ce test confirme que la tension de la résistance du capteur **31** est sensible à la pression. Si la pression varie, une variation de la tension de résistance se produira qui entrainera un changement de température du capteur d'hydrogène. Cela se traduira par la modification de la sensibilité du capteur **31** d'hydrogène, et donc un changement dans la valeur de fuite détectée. En raison de cette sensibilité, le circuit d'équilibrage de la tension a été réalisé de façon à ce que le capteur **31** soit toujours chauffé à la même température quelle que soit la pression.

Dans un deuxième temps, on a évalué la sensibilité du capteur **31** d'hydrogène à différentes pressions P dans la ligne de vide et pour différents taux de fuite. La figure 6 illustre la variation de tension ΔV dans le temps, en mV / s, en fonction de plusieurs valeurs du taux de fuite L. Le test a été réalisé aux pressions P suivantes dans la ligne de vide:

| | |
|---|---|
| P = 850 mbar | courbe 50 |
| P = 500 mbar | courbe 51 |
| P = 200 mbar | courbe 52 |
| P = 100 mbar | courbe 53 |
| P = 50 mbar | courbe 54 |
| P = 20 mbar | courbe 55 |
| P = 10 mbar | courbe 56 |
| P = 5 mbar | courbe 57 |

Le gaz traceur contenant de l'hydrogène, représentatif d'une fuite, est injecté dans le dispositif de test **30** à une vitesse imposée par la microvanne **35** qui a été préalablement calibrée. Lorsque la microvanne **35** et les vanne d'isolement **36, 37** sont ouvertes, le flux de gaz traceur entre en contact avec le capteur **31** d'hydrogène et la tension du transistor du capteur **31** d'hydrogène diminue. Lorsque la microvanne **35** et les vanne d'isolement **36, 37** sont fermées, le flux de gaz traceur est interrompu et la tension du transistor du capteur **31** d'hydrogène augmente. Les variations de la pente de la courbe d'augmentation et de diminution de la tension sont mesurées et analysées afin de déterminer un taux de fuite.

Le test a été effectué de la manière suivante. L'alimentation du capteur est mise en route. Le capteur est alimenté sous une tension de diode de 0,574 V et un courant de transistor de 0,1mA, ce qui correspond à une température de 130°C. Pendant toute la durée du test, la tension de sortie de la diode, représentative de la stabilité de la température, et la tension de sortie du transistor sont mesurées et enregistrées. La tension de sortie de la diode est restée stable pendant le test, ce qui traduit une excellente maîtrise de la température du capteur d'hydrogène. L'observation des courbes obtenues montre que le capteur d'hydrogène est le plus sensible aux plus basses pressions, notamment à 10 mbar. On voit aussi que la sensibilité du capteur d'hydrogène diminue avec la valeur du taux de fuite, elle est meilleure pour des taux de fuite élevés.

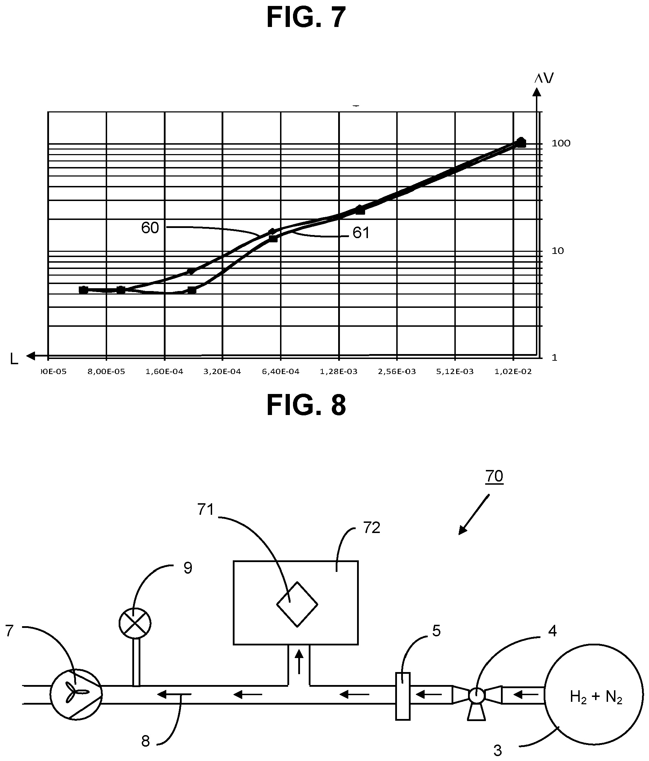

Dans un troisième temps, un test a été effectué pour identifier l'influence de la distance entre le capteur d'hydrogène et la fuite. La figure 7 illustre le résultat obtenu. La courbe **60** de variation de tension en fonction du taux de fuite à une distance de 35 cm est très semblable à la courbe **61** obtenue à une distance de 94 cm. La sensibilité du capteur d'hydrogène est donc peu influencée par la position de la fuite.

Dans un quatrième temps, on s'est intéressé à l'influence de la position du capteur d'hydrogène dans le dispositif de détection sur sa sensibilité. La figure 8 illustre le dispositif **70** utilisé pour ce test. Ce dispositif de test **70** est analogue au dispositif de détection de la figure 1 à l'exception du fait que le capteur **71** d'hydrogène et l'enceinte basse pression **72** qui le contient sont placés sur un conduit en dérivation par rapport au conduit principal. La tête du capteur **71** d'hydrogène ne se trouve donc plus face au flux principal de gaz **8** contenant de l'hydrogène issu de l'objet à tester **3** et pompé par la pompe à vide **7.**

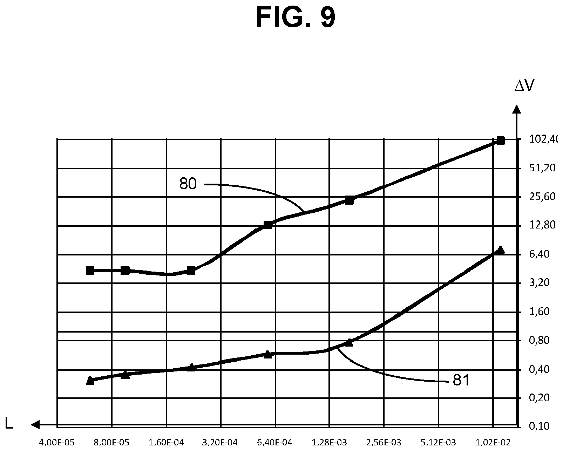

La figure 9 montre les courbes **80** et **81** correspondants respectivement à la position du capteur **2** d'hydrogène sur la figure 1 et à la position du capteur **71** sur la figure 8. Ce résultat confirme que la sensibilité du capteur **71** d'hydrogène de la figure 8 est moindre que celle du capteur **2** de la figure 1. Ce résultat confirme que le capteur d'hydrogène doit être placé de préférence dans une position telle que sa tête se trouve face au flux principal de gaz **8** contenant de l'hydrogène issu de l'objet à tester **3** et pompé par le moyen de pompage **7.**

Bien entendu, la présente invention n'est pas limitée aux modes de réalisation décrits, mais elle est susceptible de nombreuses variantes accessibles à l'homme de l'art sans que l'on s'écarte de l'esprit de l'invention. En particulier, la vanne multivoies peut être une vanne à trois voies, mais pourrait aussi être remplacée par deux vannes, chacune à deux voies par exemple, ou par tout autre moyen configuré pour mettre en communication au moins trois voies, voire plus de trois voies. En outre l'enceinte basse pression dans laquelle est placé le capteur peut prendre la forme d'une chambre à vide, par exemple mécano-soudée en inox ou en aluminium, d'une portion de canalisation fermée de manière étanche, ou d'une cavité ménagée au sein d'un bloc de matière, par exemple par usinage, ou encore de tout autre volume fermé de manière suffisamment étanche pour supporter une pression sensiblement plus basse que la pression régnant dans son environnement immédiat.

## Revendications

1. Dispositif de détection de fuite (1) au moyen d'hydrogène comme gaz traceur, destiné à être relié à un objet à tester (3), **caractérisé en ce qu'**il comprend :
- un capteur d'hydrogène (2, 20), placé dans une enceinte basse pression (6) comportant une diode (21), une résistance (22), et un transistor (23) de type MOS dont la grille (24) est recouverte d'un catalyseur au palladium,
- un moyen de pompage (7) relié à l'enceinte basse pression (6),
- une jauge de pression (9), configurée pour mesurer la pression dans une ligne de vide formée par l'enceinte basse pression reliée au moyen de pompage,
- une vanne multivoies (4) comprenant une première voie permettant l'admission du flux gazeux contenant le gaz traceur dans la ligne de vide et une deuxième voie d'admission de gaz neutre,
et **caractérisé en ce qu'**il comporte un module de contrôle électronique de la vanne multivoies (4) configuré pour contrôler la pression de la ligne de vide en fonction de la jauge de pression (9) par le contrôle de l'admission de gaz neutre dans la ligne de vide à travers la deuxième voie d'admission.

2. Dispositif de détection de fuite selon la revendication 1, dans lequel la deuxième voie est configurée pour s'ouvrir sur l'atmosphère extérieure.

3. Dispositif de détection de fuite selon l'une des revendications 1 à 2, comprenant en outre un filtre (5) à particules interposé entre le capteur d'hydrogène (2, 20) et la vanne multivoies (4) pour filtrer les particules de poussière contenues dans le gaz avant que celui-ci n'entre en contact avec le capteur d'hydrogène (2, 20).

4. Dispositif de détection de fuite selon l'une des revendications précédentes, dans lequel ledit module de contrôle électronique est configuré pour recevoir la valeur de la pression de la ligne de vide mesurée par la jauge de pression (9), configuré pour recevoir une valeur consigne et configuré pour piloter l'admission de gaz neutre en fonction de ces valeurs.

5. Dispositif de détection de fuite selon l'une des revendications précédentes, comprenant en outre un modèle mathématique pour calculer le taux de fuite à partir de la variation de la tension drain - source du transistor contenu dans le capteur d'hydrogène (2, 20) en fonction du temps.

6. Dispositif de détection de fuite selon l'une des revendications précédentes, comprenant en outre un circuit d'équilibrage de la tension de la résistance du capteur.

7. Dispositif de détection de fuite selon la revendication 6, dans lequel le circuit d'équilibrage de la tension de la résistance du capteur est configuré pour maintenir une température fixe indépendamment de la valeur de la pression de la ligne de vide.

8. Dispositif de détection de fuite selon la revendication 7, dans lequel la température fixe est comprise entre 100[deg.]C et 250[deg.]C.

9. Dispositif de détection de fuite selon l'une des revendications précédentes, dans lequel la pression dans l'enceinte (6) basse pression est comprise entre 100 Pa et 5000 Pa.

10. Procédé de détection de fuite d'un objet à tester au moyen d'un dispositif de détection de fuite selon l'une des revendications précédentes, dans lequel on contrôle la pression de la ligne de vide en contrôlant l'admission du gaz neutre dans la ligne de vide à travers la deuxième voie d'admission de la vanne multivoie.

11. Procédé de détection de fuite selon la revendication 10, dans lequel on contrôle l'admission du gaz neutre en pilotant la deuxième voie de manière automatique en fonction de la mesure de la pression et de la valeur de la consigne prédéterminée.

## Patentansprüche

1. Vorrichtung zum Erkennen von Lecks (1) mithilfe von Wasserstoff als Spurgas, welche dazu bestimmt ist, mit einem Prüfobjekt (3) verbunden zu werden, **dadurch gekennzeichnet, dass** sie umfasst:
- einen in einem Niederdruckraum (6) angeordneten Wasserstoffsensor (2, 20), der eine Diode (21), einen Widerstand (22) und einen Transistor (23) vom MOS-Typ, dessen Gate (24) mit einem Palladiumkatalysator beschichtet ist, aufweist,
- ein Pumpmittel (7), das mit dem Niederdruckraum (6) verbunden ist,
- einen Druckmesser (9), der dafür ausgelegt ist, den Druck in einer von dem Niederdruckraum gebildeten Vakuumleitung zu messen, die mit dem Pumpmittel verbunden ist,
- ein Mehrwegeventil (4), das einen ersten Weg, der den Einlass des das Spurgas enthaltenden Gasstroms in die Vakuumleitung ermöglicht, und einen zweiten Weg für den Einlass von Neutralgas umfasst,
und **dadurch gekennzeichnet, dass** sie ein Modul zur elektronischen Steuerung des Mehrwegeventils (4) umfasst, das dafür ausgelegt ist, den Druck der Vakuumleitung in Abhängigkeit von dem Druckmesser (9) durch die Steuerung des Einlasses von Neutralgas über den zweiten Einlassweg in die Vakuumleitung zu steuern.

2. Vorrichtung zum Erkennen von Lecks nach Anspruch 1, wobei der zweite Weg dafür ausgelegt ist, sich zur äußeren Atmosphäre hin zu öffnen.

3. Vorrichtung zum Erkennen von Lecks nach einem der Ansprüche 1 bis 2, welche außerdem ein Partikelfilter (5) umfasst, das zwischen dem Wasserstoffsensor (2, 20) und dem Mehrwegeventil (4) angeordnet ist, um die Staubpartikeln herauszufiltern, die in dem Gas enthalten sind, bevor dieses mit dem Wasserstoffsensor (2, 20) in Kontakt kommt.

4. Vorrichtung zum Erkennen von Lecks nach einem der vorhergehenden Ansprüche, wobei das elektronische Steuermodul dafür ausgelegt ist, den von dem Druckmesser (9) gemessenen Wert des Druckes der Vakuumleitung zu empfangen, dafür ausgelegt ist, einen Einstellwert zu empfangen, und dafür ausgelegt ist, den Einlass von Neutralgas in Abhängigkeit von diesen Werten vorzusteuern.

5. Vorrichtung zum Erkennen von Lecks nach einem der vorhergehenden Ansprüche, welche außerdem ein mathematisches Modell zum Berechnen der Leckrate aus der Änderung der Drain-Source-Spannung des in dem Wasserstoffsensor (2, 20) enthaltenen Transistors in Abhängigkeit von der Zeit umfasst.

6. Vorrichtung zum Erkennen von Lecks nach einem der vorhergehenden Ansprüche, welche außerdem eine Ausgleichsschaltung für die Spannung über dem Widerstand des Sensors umfasst.

7. Vorrichtung zum Erkennen von Lecks nach Anspruch 6, wobei die Ausgleichsschaltung für die Spannung über dem Widerstand des Sensors dafür ausgelegt ist, eine feste Temperatur unabhängig vom Wert des Druckes der Vakuumleitung aufrechtzuerhalten.

8. Vorrichtung zum Erkennen von Lecks nach Anspruch 7, wobei die feste Temperatur zwischen 100 °C und 250 °C liegt.

9. Vorrichtung zum Erkennen von Lecks nach einem der vorhergehenden Ansprüche, wobei der Druck in dem Niederdruckraum (6) zwischen 100 Pa und 5000 Pa liegt.

10. Verfahren zum Erkennen von Lecks eines Prüfobjekts mittels einer Vorrichtung zum Erkennen von Lecks nach einem der vorhergehenden Ansprüche, wobei der Druck der Vakuumleitung gesteuert wird, indem der Einlass des Neutralgases in die Vakuumleitung über den zweiten Einlassweg des Mehrwegeventils (4) gesteuert wird.

11. Verfahren zum Erkennen von Lecks nach Anspruch 10, wobei der Einlass des Neutralgases gesteuert wird, indem der zweite Weg in Abhängigkeit von der Messung des Drucks und von dem vorbestimmten Einstellwert automatisch vorgesteuert wird.

## Claims

1. Device (1) for leak detection by means of hydrogen as a tracer gas, which device is intended to be connected to an object (3) to be tested and is **characterized in that** it comprises:
- a hydrogen sensor (2, 20) placed in a low-pressure compartment (6) and comprising a diode (21), a resistor (22) and a MOS-type transistor (23) whose gate (24) is covered with a palladium catalyst,
- a pumping means (7) connected to the low-pressure compartment (6),
- a pressure gauge (9) configured to measure the pressure in a vacuum line formed by the low-pressure compartment connected to the pumping means,
- a multiway valve (4) comprising a first port allowing admission of the gas flow containing the tracer gas into the vacuum line, and a second port for the admission of neutral gas,
and **characterized in that** it includes a module for the electronic control of the multiway valve (4) which is configured to control the pressure in the vacuum line as a function of the pressure gauge (9) by controlling the admission of neutral gas into the vacuum line through the second admission port.

2. Leak detection device according to Claim 1, wherein the second port is configured to be open to the external atmosphere.

3. Leak detection device according to either of Claims 1 and 2, furthermore comprising a particle filter (5) interposed between the hydrogen sensor (2, 20) and the multiway valve (4) in order to filter the dust particles contained in the gas before it enters into contact with the hydrogen sensor (2, 20).

4. Leak detection device according to one of the preceding claims, wherein said electronic control module is configured to receive the value of the pressure of the vacuum line measured by the pressure gauge (9), is configured to receive a setpoint value and is configured to control the admission of neutral gas as a function of these values.

5. Leak detection device according to one of the preceding claims, furthermore comprising a mathematical model for calculating the leakage rate on the basis of the variation of the drain-source voltage of the transistor contained in the hydrogen sensor (2, 20) as a function of time.

6. Leak detection device according to one of the preceding claims, furthermore comprising a circuit for balancing the voltage of the resistor of the sensor.

7. Leak detection device according to Claim 6, wherein the circuit for balancing the voltage of the resistor of the sensor is configured to maintain a fixed temperature independently of the value of the pressure of the vacuum line.

8. Leak detection device according to Claim 7, wherein the fixed temperature lies between 100°C and 250°C.

9. Leak detection device according to one of the preceding claims, wherein the pressure in the low-pressure compartment (6) lies between 100 Pa and 5000 Pa.

10. Method for leak detection of an object to be tested by means of a leak detection device according to one of the preceding claims, wherein the pressure of the vacuum line is controlled by controlling the admission of the neutral gas into the vacuum line through the second admission port of the multiway valve.

11. Method for leak detection according to Claim 10, wherein the admission of the neutral gas is controlled by controlling the second port automatically as a function of the measurement of the pressure and the value of the predetermined setpoint.
